# EUROPÄISCHE PATENTSCHRIFT

(11) **EP 2 904 993 B1**
(45) Veröffentlichungstag und Bekanntmachung des Hinweises auf die Patenterteilung: **21.12.2016**
(21) Anmeldenummer: 15153312.2
(22) Anmeldetag: 30.01.2015
(51) Int. Cl.: A61F 5/37, A61F 5/01

(54) **Orthese zur Klumpfußbehandlung**
Orthosis for treating clubfoot
Orthèse destinée à traiter un pied-bot

(30) Priorität: 10.02.2014 DE 202014001080 U
(43) Veröffentlichungstag der Anmeldung: 12.08.2015
(73) Patentinhaber: Semeda Medizinische Instrumente e.K., 29389 Bad Bodenteich (DE)
(72) Erfinder: Sinclair, Marc, Al Safa 2, Dubai (AE); Kujus, harald, 29413 Dähre (DE)
(74) Vertreter: Hauck Patentanwaltspartnerschaft mbB

(56) Entgegenhaltungen:
- EP-A1- 1 998 726
- DE-A1-102012 006 261
- US-A- 4 157 876
- US-A- 5 346 463
- US-A1- 2008 214 975

## Beschreibung

Die Erfindung bezieht sich auf eine Orthese zur Klumpfußbehandlung.

Bekannt ist die Behandlung des Klumpfußes durch Gipsredressionstherapie. Der Fuß wird gerichtet und ein Gips an den gerichteten Fuß angelegt. Dies erfolgt in einigen Schritten, wobei der Fuß immer weiter in Korrekturrichtung geformt wird.

Ferner bekannt sind Orthesen zur konservativen Klumpfußbehandlung nach Ponseti. Bei einer bekannten Orthese aus starrem Kunststoff sind zwei parallele Stangen in einer Klemmvorrichtung einstellbar fixiert. Die Stangen haben an ihren äußeren Enden festklemmbare Gelenke, die mit Platten verbunden sind. Die Platten sind lösbar mit Schuhen des Patienten verbindbar. Die Gelenke ermöglichen ein Einstellen und Festlegen der Füße im geforderten therapeutischen Bereich der Dorsalextensiön (Anheben des Fußes zum Körper) von 0° bis 20° und der Abduktion (Schwenken der Füße nach außen) im Bereich von 0° bis 80°. Die Gelenke werden in eine bestimmte Schwenkstellung eingestellt und in dieser Stellung mittels einer Klemmschraube fixiert.

Die Therapie beginnt direkt nach der Geburt mit der Gipsredressionstherapie. Einige Wochen später wird die Orthese angewandt. Die Therapie endet etwa mit dem vierten Lebensjahr. Das Tragen der Orthese stellt eine erhebliche Belastung für den Säugling beziehungsweise das Kleinkind dar, da sie dessen Beweglichkeit stark einschränkt.

Aus der US 4,157,876 ist eine Orthese mit Gelenken bekannt, die in den eingestellten Schwenkpositionen mittels Klemmverschraubungen fixiert,werden.

Aus der US 3,477,426, US 4,040,416, DE 27 49 411 A1 und US 5,364,463 sind Orthesen bekannt, bei denen die Verbindungsstrukturen zwischen den Gelenken beziehungsweise die Befestigungseinrichtungen für die Füße insgesamt elastisch ausgeführt sind. Hierdurch werden große Verformungen der Orthesen ermöglicht, was die Belastungen des Patienten vermindert. Dabei verlassen die Füße die therapeutisch gewünschte Ausrichtung.

Die EP 1 989 726 B1 beschreibt eine Orthese zur Klumpfußbehandlung, die die Belastung des Patienten mindert und dennoch die Füße des Patienten in der gewünschten Ausrichtung fixiert. Hierzu weist die Orthese gemäß einer Variante mindestens einen elastischen Abschnitt auf der von einer Halteeinrichtung für die Füße abgewandten Seite mindestens eines Gelenkes zwischen diesem Gelenk und einem weiteren Abschnitt der Verbindungsstruktur auf. Gemäß einer anderen Variante ist mindestens ein elastischer Abschnitt zwischen mindestens einem Gelenk und einer Halteeinrichtung für die Füße angeordnet.

Die US 2007/0088240 A1 beschreibt eine Orthese, die die Füße des Patienten in einem gewünschten Winkel hält, wobei sie dem Patienten eine Bewegung in einer sonst normalen Weise ermöglicht und Blasenbildung vermeidet. Die Orthese weist Gelenke auf, die durch Schwenken der Füße in der Transversalebene beziehungsweise um vertikale Achsen und ein Kippen der Füße in der Frontalebene um horizontale Achsen ermöglicht. In der vorliegenden Anmeldung beziehen sich die vertikalen und horizontalen Ausrichtungen der Achsen auf einen Patienten, der mit der Orthese an den Füßen auf dem Boden steht. Die Gelenke, die ein Schwenken der Füße um die vertikalen Achsen ermöglichen, sind in einer gewünschten Schwenkposition feststellbar. Die Gelenke, die ein Schwenken um horizontale Achsen ermöglichen, sind frei beweglich. Bei einer kommerzialisierten Ausführung dieser Orthesen sind in die letzt genannten Gelenke Schenkelfedern integriert, die die Füße in eine leicht angehobene Stellung vorspannen. Diese Gelenke weisen Lagerhülsen auf, die mit Platten zum Halten der Füße verbunden sind und auf Schrauben schwenkbar gelagert sind, die in Bohrungen in den Enden des plattenförmigen Verbindungsträgers gehalten sind. Auf den Schrauben sind im Inneren der Lagerhülsen Schraubenfedern geführt, die mit einem Schenkel in ein kleines Loch des Verbindungsträgers mit einem anderen Schenkel in eine Aussparung der Lagerhülse eingreift. Bei dieser Konstruktion können durch extremes Schwenken der Gelenke die Federschenkel abgeschert werden. Aufgrund des weiten Schwenkbereichs besteht die Möglichkeit, dass das Kind seine Füße in die Spreitzfußposition bringt, wodurch die Gefahr eines Spreitzfußrezidivs besteht. Außerdem besteht aufgrund der offenen Bauweise der Gelenke Verletzungsgefahr. Ferner schränkt die Bauweise die Möglichkeit, die Feder durch eine Feder mit anderen Federkennwerten zu ersetzen, stark ein.

Die DE 10 2012 006 261 A1 beschreibt eine Orthese gemäß dem Oberbegriff des Anspruchs 1, zur Klumpfußbehandlung mit einem Verbindungsträger, Mitteln zum lösbaren Halten der Füße eines Patienten und Gelenken, über die die Mittel zum lösbaren Halten der Füße mit verschiedenen Enden des Verbindungsträgers verbunden sind. Die Gelenke ermöglichen ein Schwenken der von den Mitteln zum lösbaren Halten gehaltenen Füße um horizontale Achsen. Sie weisen jeweils einen Lagerträger auf, der mit einem Ende des Verbindungsträgers verbunden ist. In einer Lagerbohrung des Lagerträgers ist eine Welle drehbar gelagert. Auf der anderen Seite des Lagerträgers ist ein Rotor angeordnet, der drehfest mit der Welle verbunden und mit den Mitteln zum lösbaren Halten eines Fußes verbunden ist. Die Welle ist auf der anderen Seite des Lagerträgers drehfest mit einem radial vorstehenden Exzenter und Mitteln zum Befestigen einer Feder verbunden. Auf derselben Seite weist der Lagerträger weitere Mittel zum Befestigen einer Feder auf. An den beiden Mitteln zum Befestigen ist eine Feder befestigt. Bei dieser Orthese ist das Versagens- und Verletzungsrisiko vermindert und die Bestückung der Gelenke mit unterschiedlichen Federn erleichtert.

Davon ausgehend liegt der Erfindung die Aufgabe zugrunde, eine Orthese zur Klumpfußbehandlung zu schaffen, die eine stabile Bauweise hat, mit wenigen Bauteilen auskommt und einfach montierbar ist.

Die Aufgabe wird durch eine Orthese mit den Merkmalen von Anspruch 1 gelöst.

Die erfindungsgemäße Orthese zur Klumpfußbehandlung hat
- ein langgestrecktes Trägerelement,
- Mittel zum Halten der Füße eines Patienten und
- ein Gelenk an jedem Ende des Trägerelements, über das jeweils ein Mittel zum Halten eines Fußes mit dem Trägerelement verbunden ist,
dadurch gekennzeichnet, dass
- das Gelenk ein Gehäuse mit zwei Gehäusehälften aufweist, die über Verbindungsmittel miteinander verbunden sind,
- im Gehäuse ein Schwenkkörper angeordnet ist, der über Lagermittel an mindestens einer Gehäusehälfte schwenkbar gelagert ist,
- das Gehäuse eine Gehäuseöffnung aufweist, die sich in einer Richtung senkrecht zur Schwenkachse des Schwenkkörpers erstreckt,
- der Schwenkkörper über ein Verbindungselement, das sich durch die Gehäuseöffnung hindurch erstreckt, mit dem außerhalb des Gehäuses angeordneten Mittel zum lösbaren Halten eines Fußes verbunden ist und
- ein Federelement im Gehäuse angeordnet ist, das an einem Widerlager am Schwenkkörper und an einem weiteren Widerlager am Gehäuse angreift.

Die erfindungsgemäße Orthese hat durch die Lagerung des Schwenkkörpers in einem Gehäuse mit zwei Gehäusehälften und das am Schwenkkörper und am Gehäuse angreifende Federelement einen besonders einfachen und robusten Aufbau und ist einfach montierbar.

Sie ist leicht mit einem Federelement mit Federkennwerten bestückbar, die für die jeweilige Behandlung ausgewählt werden können. Sie kann leicht für die jeweilige Behandlung vorbereitet werden. Im Gebrauch ist sie vorteilhaft, insbesondere wegen ihres geringem Gewichts und der raumsparenden Bauweise.

Vorzugsweise sind die Gelenke so beschaffen, dass die von den Mitteln zum lösbaren Halten gehaltenen Füße um horizontale Achsen schwenkbar sind.

Das Gehäuse kann durch die Gehäusehälften in gleichgroße oder verschiedengroße Teile unterteilt sein. Gemäß einer bevorzugten Ausgestaltung der Erfindung ist das Gehäuse in annähernd gleichgroße Gehäusehälften unterteilt. Gemäß einer weiteren Ausgestaltung sind die Gehäusehälften schalenförmig.

Gemäß einer weiteren Ausgestaltung weisen die Gehäusehälften an der Innenseite jeweils ein Achsstück auf und ist der Schwenkkörper mit mindestens einer Lagerhülse auf den beiden Achsstücken gelagert. Gemäß einer weiteren Ausgestaltung weist der Schwenkkörper zwei Lagerhülsen auf, von denen jede auf einem Achsstück einer Gehäusehälfte gelagert ist. Gemäß einer weiteren Ausgestaltung ist in jeder Lagerhülse eine Lagerbuchse angeordnet, die auf einem Achsstück gelagert ist. Gemäß einer alternativen Ausführung weist der Schwenkkörper Achsstücke auf, die in Lagerlöchern der Gehäusehälften gelagert sind.

Gemäß einer weiteren Ausgestaltung weist der Lagerkörper an beiden Stirnseiten eines hohlzylindrischen Grundkörpers jeweils eine Lagerhülse auf. Der hohlzylindrische Grundkörper ist vorzugsweise ein Segment eines Hohlzylinders. Hierdurch wird eine besonders materialsparende und leichte Ausführung des Lagerkörpers ermöglicht.

Gemäß einer weiteren Ausgestaltung steht das Verbindungselement von der Außenseite des hohlzylindrischen Grundkörpers vor und überdeckt der hohlzylindrische Hohlkörper die Gehäuseöffnung innen blendenartig. Bei dieser Ausgestaltung deckt der hohlzylindrische Grundkörper die Gehäuseöffnung innen ab, wodurch die Gefahr des Einklemmens von Körperteilen vermieden wird.

Gemäß einer weiteren Ausgestaltung ist das Federelement eine Schenkelfeder (oder Drehfeder), die sich mit einem Schenkel am Widerlager des Schwenkkörpers und mit einem weiteren Schenkel am gehäusefesten Widerlager abstützt.

Gemäß einer weiteren Ausgestaltung ist das Widerlager des Schwenkkörpers ein Stift, der beidenends in Löchern in den beiden Lagerhülsen das Lagerkörpers gehalten ist. Gemäß einer weiteren Ausgestaltung ist das gehäusefeste Widerlager ein weiterer Stift, der beidenends an Löchern in den beiden Gehäusehälften gehalten ist.

Gemäß einer weiteren Ausgestaltung weist das Gehäuse an einem Ende eine teilzylindrische Form mit einem großen Durchmesser und am anderen Ende eine teilzylindrische Form mit einem kleinen Durchmesser, im Vergleich zu der teilzylindrischen Form mit dem großen Durchmesser auf, wobei die beiden teilzylindrischen Formen unten durch eine flache, im Wesentlichen horizontale Bodenwand des Gehäuses miteinander verbunden sind und die beiden teilzylindrischen Formen oben durch eine im Wesentlichen flache, zur Horizontalen geneigte Deckwand des Gehäuses miteinander verbunden sind. Der Schwenkkörper ist im Wesentlichen im Bereich der teilzylindrischen Form mit dem großen Durchmesser angeordnet und das Federelement ist im Wesentlichen im Bereich der teilzylindrischen Form mit dem kleinen Durchmesser angeordnet. Diese Ausgestaltung ist für eine platzsparende Unterbringung und einfache Montage und Demontage des Gelenks vorteilhaft.

Gemäß einer weiteren Ausgestaltung erstreckt sich die Gehäuseöffnung in beide Gehäusehälften. Dies ist vorteilhaft für eine stabile Ausführung des Verbindungselementes.

Gemäß einer weiteren Ausgestaltung ist das Verbindungselement pfostenförmig.

Gemäß einer weiteren Ausgestaltung weist das Gehäuse an einem Ende ein Langloch auf, das sich in beide Gehäusehälften erstreckt und ein im Wesentlichen streifenförmiges Ende des Trägerelements aufnimmt, wobei das streifenförmige Ende des Trägerelements und mindestens eine Gehäusehälfte zusammenwirkende Verriegelungsmittel aufweisen, die das Trägerelement mit dem Gehäuse verriegeln und durch Lösen der Gehäusehälften voneinander entriegelbar sind. Hierdurch wird eine besonders stabile und sichere Fixierung des Gelenks am Trägerelement erreicht, die einfach montierbar und demontierbar ist.

Gemäß einer weiteren Ausgestaltung ist das Langloch in dem Ende des Gehäuses mit der großen teilzylindrischen Form ausgebildet. Dies begünstigt eine platzsparende Fixierung des Gelenks am Trägerelement.

Gemäß einer weiteren Ausgestaltung sind die Gehäusehälften durch Verbindungsmittel miteinander verbunden, die mindestens ein Schraubenloch in einer Gehäusehälfte, mindestens ein Innengewinde in einer anderen Gehäusehälfte und eine in das Schraubenloch und das Innengewinde eingeschraubte und die Gehäusehälften miteinander verbindende Schraube umfassen.

Gemäß einer weiteren Ausgestaltung umfasst die Orthese mindestens einen Arretierstift und weisen der Schwenkkörper und das Gehäuse jedes Gelenks jeweils ein Arretierloch auf, die in einer bestimmten Schwenkstellung des Schwenkkörpers zum Gehäuse miteinander fluchten, wobei der Arretierstift bei geöffnetem Gehäuse in die Arretierlöcher einsetzbar ist und bei geschlossenem Gehäuse den Schwenkkörper in der bestimmten Schwenkstellung fixiert. Dies ermöglicht ein einfaches Fixieren bzw. Festhalten des Schwenkkörpers in der bestimmten Schwenkstellung, das bei manchen Therapien von Vorteil sein kann.

Gemäß einer weiteren Ausgestaltung sind der Schwenkkörper, das Verbindungselement und ein Lagerelement für die Mittel zum lösbaren Halten an einem vom Schwenkkörper entfernten Ende des Verbindungselementes einteilig ausgebildet. Dies ist vorteilhaft für Stabilität, Montage und Demontage der Orthese.

Gemäß einer weiteren Ausgestaltung weist jedes Mittel zum lösbaren Halten eines Fußes eine Fußplatte mit Mitteln zum Befestigen eines Fußes oder eines Schuhes auf.

Gemäß einer weiteren Ausgestaltung ist die Fußplatte in verschiedenen Schwenkstellungen am Lagerelement fixierbar. Hierdurch ist bei jeder Therapie eine individuelle Einstellung der Ausrichtung der Fußplatte möglich. Gemäß einer bevorzugten Ausgestaltung ist das Lagerelement eine Verbindungsplatte vorzugsweise in Form einer Kreisscheibe. Gemäß einer weiteren Ausgestaltung weisen das Lagerelement und/oder die Fußplatte eine Markierung zur Anzeige der Schwenkstellung der Fußplatte bezüglich des Lagerelementes auf. Gemäß einer weiteren Ausgestaltung weisen die Fußplatte und/oder das Lagerelement eine Riffelung zum Fixieren der Fußplatte in einer bestimmten Schwenkstellung am Lagerelement auf. Gemäß einer weiteren Ausgestaltung ist die Fußplatte mittels einer Schraube in einer bestimmten Schwenkstellung am Lagerelement fixierbar.

Gemäß einer bevorzugten Ausgestaltung ist das Gehäuse ganz oder teilweise aus Kunststoff hergestellt. Vorzugsweise ist das Gehäuse aus Kunststoff spritzgegossen. Weiterhin vorzugsweise ist das Innengewinde in einem Metalleinsatz ausgebildet, der im Gehäuse gehalten ist. Vorzugsweise ist der Metalleinsatz mit dem Material des Gehäuses umspritzt. Alternativ ist das Innengewinde direkt in dem Kunststoffmaterial des Gehäuses ausgebildet.

Gemäß einer weiteren Ausgestaltung ist der Schwenkkörper aus Kunststoff hergestellt. Weiterhin vorzugsweise besteht er aus glasfaserverstärktem Kunststoff. Vorzugsweise bestehen die Lagerbuchsen aus einem Metall.

Weiterhin vorzugsweise ist das Federelement aus einem Federstahl hergestellt.

Die Schraube zum Verbinden der Gehäusehälften ist vorzugsweise eine Metallschraube.

In der vorliegenden Anmeldung beziehen sich die Angaben "oben" und "unten" auf eine Anordnung der Orthese unter den Füßen eines aufrecht stehenden Menschen. Dies gilt auch für die Angaben "horizontal" und "vertikal".

Die Erfindung wird nachfolgend anhand der anliegenden Zeichnungen eines Ausführungsbeispiels näher erläutert. In den Zeichnungen zeigen:
- Fig. 1: die Orthese in einer Perspektivansicht schräg von oben und von der Vorderseite;
- Fig. 2: ein Gelenk der Orthese mit einem Ende des Trägerelementes in einem perspektivischen Sprengbild;
- Fig. 3: ein Gelenk der Orthese fixiert an einem Trägerelement in einem Vertikalschnitt.

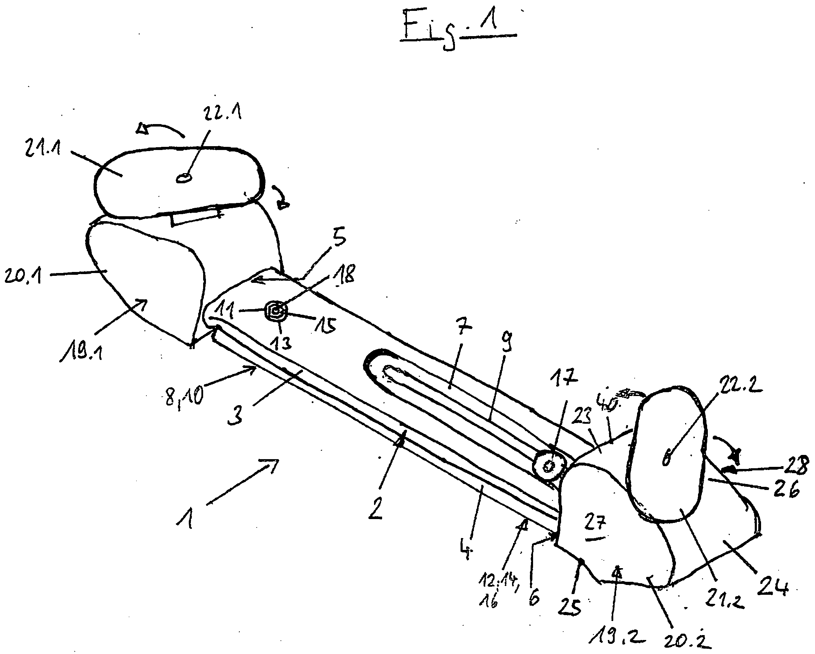

Gemäß Fig. 1 weist die Orthese 1 ein langgestrecktes Trägerelement 2 auf, das zwei aneinander geführten Leisten 3, 4 aufweist, die voneinander abgewandte, streifenförmige Enden 5, 6 aufweisen. Jede Leiste 3, 4 weist in einer länglichen Vertiefung 7, 8 einen Längsschlitz 9, 10 und ein Schraubenloch 11, 12 auf. Die Vertiefung 7, 8 und der Längsschlitz 9, 10 erstrecken sich von der Nähe eines streifenförmigen Endes 5, 6 bis über die Mitte der jeweiligen Leiste 3, 4 hinaus. Das Schraubenloch 11, 12 ist in der Nähe des anderen laschenförmigen Endes 5, 6 angeordnet. Jedes Schraubenloch 11, 12 hat auf einer Seite eine Vertiefung 13, 14, in die eine Schraubenmutter 15, 16 eingesetzt ist. In den Längsschlitz 9, 10 jedes Streifens ist eine Schraube 17, 18 eingesetzt, die in die Mutter 15, 16 im Schraubenloch 11, 12 der anderen Leiste 3, 4 eingedreht ist. Wenn die Schrauben 17, 18 gelockert sind, sind die Leisten 3, 4 auseinanderziehbar oder zusammenschiebbar. In der gewählten Auszugsstellung sind die Leisten 3, 4 aneinander durch Festdrehen der Schrauben 17, 18 fixierbar.

Das Trägerelement 2 ist an beiden Enden mit jeweils einem Gelenk 19.1, 19.2 verbunden, das ein Gehäuse 20.1, 20.2 aufweist. Ferner ist jedes Gelenk 19.1, 19.2 mit einer Fußplatte 21.1, 21.2 verbunden, die Mittel zum Halten 22.1, 22.2 eines Fußes aufweist. Jede Fußplatte 21.1, 21.2 ist bezüglich des Gelenkes 19.1, 19.2 um eine horizontale Achse schwenkbar. Ferner ist die Schwenkstellung jeder Fußplatte 21.1, 21.2 bezüglich des Trägerelementes 2 einstellbar.

Einzelheiten der Gelenke 19.1, 19.2 werden nachstehend unter Verwendung derselben Bezugsziffern für beide Gelenke 19.1, 19.2 erläutert.

Jedes Gehäuse 20 hat an dem Ende, das mit dem Trägerelement 2 verbunden ist, eine teilzylindrische Form 23 mit einem großen Durchmesser. Am anderen Ende hat das Gehäuse eine teilzylindrische Form 24 mit einem kleinen Durchmesser. Die beiden teilzylindrischen Formen 23, 24 sind unten durch eine flache, im Wesentlichen horizontale Bodenwand 25 des Gehäuses miteinander verbunden. Oben sind sie durch eine im Wesentlichen flache, zur Horizontalen geneigte Deckwand 26 miteinander verbunden. Die beiden Seitenwände 27, 28 des Gehäuses 20 sind eben und parallel zu einander ausgerichtet.

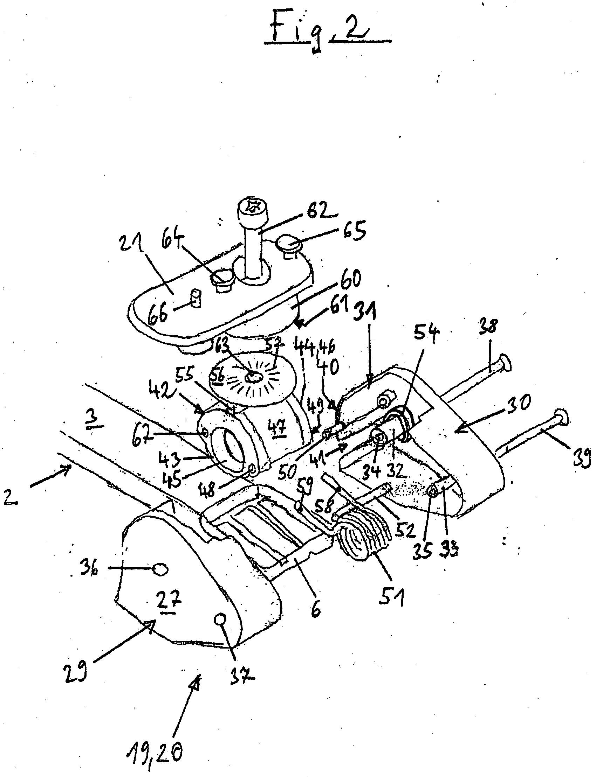

Gemäß Fig. 2 ist jedes Gehäuse 20 etwa in der Mitte in zwei schalenförmige Gehäusehälften 29, 30 geteilt. Das Gehäuse 20 hat oben eine Gehäuseöffnung 31, die sich in beide Gehäusehälften 29, 30 erstreckt.

Jede Gehäusehälfte 29, 30 hat von der Innenseite vorstehende Hülsen 32, 33, in denen Schraubenlöcher 34, 35 ausgebildet sind. Eine Gehäusehälfte 29 weist in der Seitenwand 27 Innengewinde 36, 37 auf, in die Schrauben 38, 39 einschraubbar sind, die von außen in die Schraubenlöcher 34, 35 der anderen Gehäusehälfte 30 einführbar sind.

Ferner ist in einer vorderen Wand 40 des Gehäuses, die dem Trägerelement zugewandt ist, ein Langloch 41 ausgebildet, das sich in beide Gehäusehälften 29, 30 erstreckt.

Die beiden Gehäusehälften 29, 30 sind mit den Abschnitten des Langloches 41 auf das laschenförmige Ende 5, 6 des Trägerelementes 2 aufschiebbar, wobei Verriegelungselemente am laschenförmigen Ende 5, 6 und in den Gehäusehälften 29, 30 ineinandergreifen. Die aufgeschobenen Gehäusehälften 29, 30 sind durch Zusammenschrauben mittels der Schrauben 38, 39 an den laschenförmigen Enden 5, 6 des Trägerelemetes 2 fixierbar.

Ein Schwenkkörper 42 weist zwei miteinander fluchtende Lagerhülsen 43, 44 auf, in die Lagerbuchsen 45, 46 eingesetzt sind. Die Lagerhülsen 43, 44 sind an den beiden Stirnseiten eines hohlzylindrischen Grundkörpers 47 des Schwenkkörpers 42 angeordnet. Der hohlzylindrische Grundkörper 47 hat etwa die Form eines halben Hohlzylinders.

In den beiden Lagerhülsen 43, 44 sind miteinander fluchtende Lagerlöcher 48, 49 angeordnet. In diesen ist ein Stift 50 gehalten, der ein Widerlager für eine Schenkelfeder 51 bildet.

Ein weiterer Stift 52 ist in Lagerlöchern an den Innenseiten der beiden Gehäusehälften 29, 30 einsetzbar.

Die Gehäusehälften 29, 30 weisen an den Innenseiten ihrer Seitenwände 27, 28 jeweils ein Achsstück 53, 54 auf, auf dem der Schwenkkörper 42 mit den Lagerhülsen 43, 44 schwenkbar gelagert ist. Von der Außenseite des hohlzylindrischen Grundkörpers 47 steht ein einteilig mit diesem verbundenes pfostenförmiges Verbindungselement 55 vor, auf dem wiederum ein Lagerelement 56 fixiert ist. Das Lagerelement 56 ist kreisscheibenförmig und weist auf der Oberseite eine radial gerichtete Riffelung 57 auf.

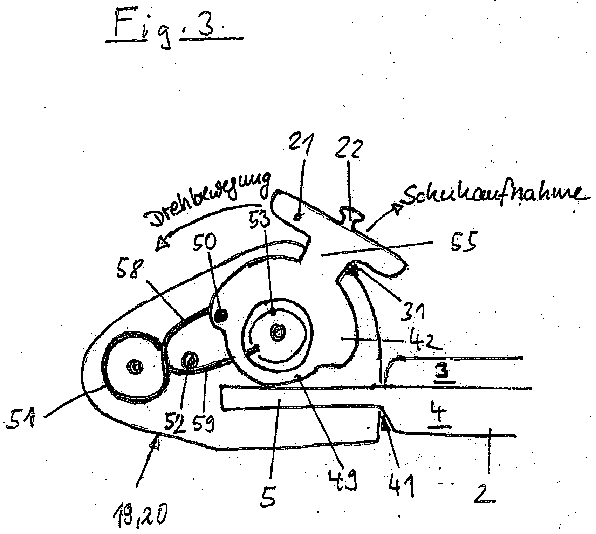

Wenn der Schwenkkörper 42 mit den Lagerhülsen 43, 44 auf den Achsstücken 53, 54 gelagert ist, durchgreift das pfostenförmige Verbindungselement 55 die Gehäuseöffnung 31. Dies ist in Fig. 3 gezeigt. Die Schenkelfeder 51 ist im Gehäuse 20 so montierbar, dass ein Schenkel 58 an dem Stift 50 angreift, der im Schwenkkörper 42 gehalten ist und der andere Schenkel 59 am Stift 52 angreift, der zwischen den Gehäusehälften 29, 30 gelagert ist.

Durch die Schenkelfeder 51 wird der Schwenkkörper 42 in einer Ausrichtung im Gelenk 20 gehalten, in der das Verbindungselement 55 etwa vertikal aus der Gehäuseöffnung 31 heraussteht. Ausgehend von dieser Lage ist der Schwenkkörper 42 in beide Richtungen schwenkbar, bis das pfostenförmige Verbindungselement 55 auf die beiden Ränder der Gehäuseöffnung 31 trifft.

Die Fußplatte 21 weist auf der Unterseite einen zylindrischen Sockel 60 auf, der mit einer radialen Riffelung 61 an der Unterseite versehen ist. Eine Schraube 62 ist durch die Fußplatte 21 und den Sockel 60 hindurchgeführt und in ein Gewindeloch 63 in der Oberseite des Lagerelements 56 eindrehbar.

Die Fußplatte 21 ist in verschiedenen Schwenkstellungen an dem Lagerelement 56 fixierbar.

Die Fußplatte 21 weist auf der Oberseite zwei vorstehende Bolzen 64, 65 mit verbreiterten Köpfen auf, die durch Aufnahmeöffnungen in Nuten in der Unterseite der Sohle eines Schuhs einführbar ist. Ferner weist die Fußplatte 21 einen verfederten Stift 66 auf, der in eine der Aufnahmeöffnungen einschnappt, wenn die Bolzenköpfe in Bereiche der Nuten eingeschoben sind, in denen sie von vorspringenden Rändern der Nuten hintergriffen werden.

Die Gelenke 19 sind vorteilhaft aus wenigen Teilen herstellbar und äußerst robust und belastbar ausführbar. Sie ermöglichen eine Ausrichtung der Füße in einem einstellbaren Winkel zur Längsachse des Trägerelementes 2. Ferner ermöglichen soe ein Schwenken der Füße um eine horizontale Ebene.

Sofern die Therapie dies erfordert, sind die Gelenke 19 auch feststellbar. Hierfür weist der Schwenkkörper 42 ein Arretierungsloc 67 und die Gehäusehälfte 29 ein weiteres Arretierungsloch auf. Bei geöffnetem Gehäuse 20 ist in die beiden Arretierungslöcher ein Arretierungsstift einsetzbar, der bei geschlossenem Gehäuse 20 den Schwenkkörper 42 an einem Schwenken im Gehäuse 20 hindert.

## Patentansprüche

1. Orthese zur Klumpfußbehandlung mit
- einem langgestreckten Trägerelement (2),
- Mitteln zum Halten (21) der Füße eines Patienten und
- einem Gelenk (19) an jedem Ende des Trägerelements (2), über das jeweils ein Mittel zum Halten (21) eines Fußes mit dem Trägerelement (2) verbunden ist,
**dadurch gekennzeichnet, dass**
- das Gelenk (19) ein Gehäuse (20) mit zwei Gehäusehälften (29, 30) aufweist, die über Verbindungsmittel (36 bis 39) miteinander verbunden sind,
- im Gehäuse (20) ein Schwenkkörper (42) angeordnet ist, der über Lagermittel (43, 44, 53, 54) an mindestens einer Gehäusehälfte (29, 30) schwenkbar gelagert ist,
- das Gehäuse (20) eine Gehäuseöffnung (31) aufweist, die sich in einer Richtung senkrecht zur Schwenkachse des Schwenkkörpers (42) erstreckt,
- der Schwenkkörper (42) über ein Verbindungselement (55), das sich durch die Gehäuseöffnung (31) hindurch erstreckt, mit dem außerhalb des Gehäuses (20) angeordneten Mittel zum lösbaren Halten (21) eines Fußes verbunden ist und
- ein Federelement im Gehäuse (20) angeordnet ist, das an einem Widerlager (50) am Schwenkkörper (42) und an einem weiteren Widerlager (52) am Gehäuse (20) angreift.

2. Orthese nach Anspruch 1, bei der die Gehäusehälften (29, 30) schalenförmig sind.

3. Orthese nach Anspruch 1 oder 2, bei der die Gehäusehälften (29, 30) an der Innenseite jeweils ein Achsstück (53, 54) aufweisen und der Schwenkkörper (42) mit mindestens einer Lagerhülse (43, 44) auf den beiden Achsstücken gelagert ist.

4. Orthese nach Anspruch 3, bei der der Lagerkörper (42) an beiden Stirnseiten eines hohlzylindrischen Grundkörpers (47) jeweils eine Lagerhülse (43, 44) aufweist.

5. Orthese nach Anspruch 4, bei der das Verbindungselement (55) von der Außenseite des hohlzylindrischen Grundkörpers (47) vorsteht und der hohlzylindrische Grundkörper (47) die Gehäuseöffnung (31) innen blendenartig überdeckt.

6. Orthese nach einem der Anspruch 1 bis 5, bei der das Federelement (51) eine Schenkelfeder ist, die sich mit einem Schenkel (58) am Widerlager (50) des Schwenkkörpers (42) und mit einem weiteren Schenkel (59) am gehäusefesten Widerlager (52) abstützt.

7. Orthese nach Anspruch 6, bei der das Widerlager (50) des Schwenkkörpers (42) ein Stift ist, der beidenends in Löchern (48, 49) in den Lagerhülsen (43, 44) gehalten ist, und das gehäusefeste Widerlager (52) ein Stift ist, der beidenends in Löchern in den beiden Gehäusehälften (29, 30) gehalten ist.

8. Orthese nach einem der Ansprüche 1 bis 7, bei der das Gehäuse (20) an einem Ende eine teilzylindrische Form (23) mit einem großen Durchmesser und am anderen Ende eine teilzylindrische Form (24) mit einem kleinen Durchmesser aufweist, wobei die beiden teilzylindrischen Formen (23, 24) unten durch eine flache, im Wesentlichen horizontale Bodenwand (25) des Gehäuses (20) miteinander verbunden sind und die beiden teilzylindrischen Formen oben durch eine im Wesentlichen flache, zur Horizontalen geneigte Deckwand (26) des Gehäuses miteinander verbunden sind, der Schwenkkörper (42) im Wesentlichen im Bereich der teilzylindrischen Form (23) mit dem großen Durchmesser angeordnet ist und das Federelement (51) im Wesentlichen im Bereich der teilzylindrischen Form (24) mit dem kleinen Durchmesser angeordnet ist.

9. Orthese nach einem der Ansprüche 1 bis 8, bei der sich die Gehäuseöffnung (31) in beide Gehäusehälften (29, 30) erstreckt.

10. Orthese nach einem der Ansprüche 1 bis 9, bei der das Gehäuse (20) an einem Ende ein Langloch (41) aufweist, das sich in beide Gehäusehälften erstreckt und ein im Wesentlichen streifenförmiges Ende des Trägerelements aufnimmt, wobei das streifenförmige Ende (5, 6) des Trägerelements (2) und mindestens eine Gehäusehälfte (29, 30) zusammenwirkende Verriegelungsmittel aufweisen, die das Trägerelement (2) mit dem Gehäuse (20) verriegeln und durch Lösen der Gehäusehälften (29, 30) voneinander entriegelbar sind.

11. Orthese nach Anspruch 8 und 10, bei der das Langloch (41) in dem Ende des Gehäuses (20) mit der großen teilzylindrischen Form (23) ausgebildet ist.

12. Orthese nach einem der Ansprüche 1 bis 11, bei der die Gehäusehälften (29, 30) durch Verbindungsmittel (36 bis 39) miteinander verbunden sind, die mindestens ein Schraubenloch in einer Gehäusehälfte (29), mindestens ein Innengewinde in einer anderen Gehäusehälfte (30) und eine in das Schraubenloch und das Innengewinde eingeschraubte und die Gehäusehälften miteinander verbindende Schraube umfassen.

13. Orthese nach einem der Ansprüche 1 bis 12, die mindestens einen Arretierstift umfasst und der Schwenkkörper (42) und das Gehäuse (20) jedes Gelenks (19) jeweils ein Arretierloch (62) aufweisen, die in einer bestimmten Schwenkstellung des Schwenkkörpers (42) zum Gehäuse (20) miteinander fluchten, wobei der Arretierstift wahlweise bei geöffnetem Gehäuse (20) in die Arretierlöcher (62) einsetzbar ist und bei geschlossenem Gehäuse (20) den Schwenkkörper (42) in der bestimmten Schwenkstellung fixiert.

14. Orthese nach einem der Ansprüche 1 bis 13, bei der der Schwenkkörper (42), das Verbindungselement (55) und ein Lagerelement (56) für die Mittel zum lösbaren Halten (21) an einem vom Schwenkkörper (42) entfernten Ende des Verbindungselementes (55) einteilig ausgebildet sind.

15. Orthese nach einem der Ansprüche 1 bis 14, bei der jedes Mittel zum lösbaren Halten (21) eines Fußes eine Fußplatte mit Mitteln zum Befestigen (22) eines Fußes oder Schuhes aufweisen.

## Claims

1. An orthosis for treating clubfoot with
- an elongated support element (2),
- means for holding (21) the feet of a patient and
- a joint (19) on each end of the support element (2), via which respectively one means for holding (21) a foot with the support element (2) is connected,
**characterized in that**
- the joint (19) has a housing (20) with two housing halves (29, 30), which are interconnected via connections means (36 to 39),
- a pivoting body (42) is arranged in the housing (20), which is pivotably mounted on at least one housing half (29, 30) via mounting means (43, 44, 53, 54),
- the housing (20) has a housing opening (31), which extends in a direction perpendicular to the pivot axis of the pivoting body (42),
- the pivoting body (42) is connected with the means for the releasable holding (21) of a foot arranged outside of the housing (20) via a connection element (55), which extends through the housing opening (31) and
- a spring element is arranged in the housing (20), which engages on a counter bearing (50) on the pivoting body (42) and on a further counter bearing (52) on the housing (20).

2. The orthosis according to claim 1, in which the housing halves (29, 30) are bowl-shaped.

3. The orthosis according to claim 1 or 2, in which the housing halves (29, 30) each have an axis piece (53, 54) on the inside and the pivoting body (42) is mounted on the two axis pieces with at least one bearing sleeve (43, 44).

4. The orthosis according to claim 3, in which the bearing body (42) has on both front sides of a hollow cylindrical base body (47) one bearing sleeve (43, 44) each.

5. The orthosis according to claim 4, in which the connection element (55) protrudes from the outside of the hollow cylindrical base body (47) and the hollow cylindrical base body (47) covers the housing opening (31) on the inside in a cover-like manner.

6. The orthosis according to one of claims 1 to 5, in which the spring element (51) is a leg spring, which is supported with one leg (58) on the counter bearing (50) of the pivoting body (42) and with a further leg (59) on the housing-fixed counter bearing (52).

7. The orthosis according to claim 6, in which the counter bearing (50) of the pivoting body (42) is a pin, which is held on both ends in holes (48, 49) in the bearing sleeves (43, 44), and the housing-fixed counter bearing (52) is a pin, which is held on both ends in holes in the two housing halves (29, 30).

8. The orthosis according to one of claims 1 to 7, in which the housing (20) has, on one end, a partially cylindrical shape (23) with a large diameter and, on the other end, a partially cylindrical shape (24) with a small diameter, wherein the two partially cylindrical shapes (23, 24) are interconnected on the bottom by a flat, mainly horizontal floor wall (25) of the housing (20) and the two partially cylindrical shapes are interconnected on the top by a cover wall (26) of the housing, which is mainly flat and tilted toward the horizontal, the pivoting body (42) is arranged mainly in the area of the partially cylindrical shape (23) with the large diameter and the spring element (51) is arranged mainly in the area of the partially cylindrical shape (24) with the small diameter.

9. The orthosis according to one of claims 1 to 8, in which the housing opening (31) extends into both housing halves (29, 30).

10. The orthosis according to one of claims 1 to 9, in which the housing (20) has an elongated hole (41) on one end, which extends into both housing halves and receives a mainly strip-shaped end of the support element, wherein the strip-shaped end (5, 6) of the support element (2) and at least one housing half (29, 30) have interacting locking means, which lock the support element (2) with the housing (20) and can be unlocked from each other by loosening the housing halves (29, 30).

11. The orthosis according to claims 8 and 10, in which the elongated hole (41) is formed in the end of the housing (20) with the large partially cylindrical shape (23).

12. The orthosis according to one of claims 1 to 11, in which the housing halves (29, 30) are interconnected through connection means (36 to 39), which comprise at least one screw hole in a housing half (29), at least one inner thread in another housing half (29), at least one inner thread in another housing half (30) and a screw screwed into the screw hole and the inner thread and interconnecting the housing halves.

13. The orthosis according to one of claims 1 to 12, which comprises at least one locking pin and the pivoting body (42) and the housing (20) of each joint (19) has a locking hole (62), which are flush with each other in a certain pivoting position of the pivoting body (42) with respect to the housing (20), wherein the locking pin is optionally insertable into the locking holes (62) when the housing (20) is open and fixes the pivoting body (42) in the specific pivoting position when the housing (20) is closed.

14. The orthosis according to one of claims 1 to 13, in which the pivoting body (42), the connection element (55) and a mounting element (56) for the means for the releasable holding (21) are designed as one piece on one end of the connection element (55) distant from the pivoting body (42).

15. The orthosis according to one of claims 1 to 14, in which each means for the releasable holding (21) of a foot has a foot plate with means for fastening (22) of a foot or shoe.

## Revendications

1. Orthèse destinée à traiter un pied-bot avec
- un élément de support étendu en longueur (2),
- des moyens pour le maintien (21) des pieds d'un patient et
- une articulation (19) à chaque extrémité de l'élément de support (2) par le biais de laquelle un moyen pour le maintien (21) d'un pied est à chaque fois connecté à l'élément de support (2),
**caractérisée en ce que**
- l'articulation (19) présente un logement (20) avec deux moitiés de logement (29, 30) qui sont connectées ensemble par le biais de moyens de connexion (36 à 39),
- un corps pivotant (32) qui est logé à pivotement par le biais de moyens de palier (43, 44, 53, 54) sur au moins une moitié de logement (29, 30) est disposé dans le logement (20),
- le logement (20) présente une ouverture de logement (31) qui s'étend dans une direction perpendiculaire à l'axe de pivotement du corps pivotant (42),
- le corps pivotant (42) est connecté au moyen disposé en dehors du logement (20) pour le maintien amovible (21) d'un pied par le biais d'un élément de connexion (55) qui s'étend à travers l'ouverture de logement (31) et
- un élément de ressort qui est appliqué sur une butée (50) sur le corps pivotant (42) et sur une autre butée (52) sur le logement (20) est disposé dans le logement (20).

2. Orthèse selon la revendication 1, dans laquelle les moitiés de logement (29, 30) sont en forme de coque.

3. Orthèse selon la revendication 1 ou 2, dans laquelle les moitiés de logement (29, 30) présentent sur le côté interne chacune une pièce axiale (53, 54) et le corps pivotant (42) est logé avec au moins un manchon de palier (43, 44) sur les deux pièces axiales.

4. Orthèse selon la revendication 3, dans laquelle le corps de palier (42) présente sur les deux côtés frontaux d'un corps de base cylindrique creux (47) à chaque fois un manchon de palier (43, 44).

5. Orthèse selon la revendication 4, dans laquelle l'élément de connexion (55) fait saillie du côté externe du corps de base cylindrique creux (47) et le corps de base cylindrique creux (47) recouvre l'ouverture de logement (31) à l'intérieur à la manière d'un écran.

6. Orthèse selon une des revendications 1 à 5, dans laquelle l'élément de ressort (51) est un ressort à branches qui s'appuie avec une branche (58) sur la butée (50) du corps pivotant (42) et avec une autre branche (59) sur la butée fixée au logement (52).

7. Orthèse selon la revendication 6, dans laquelle la butée (50) du corps pivotant (42) est une broche qui est maintenue aux deux extrémités dans des trous (48, 49) dans les manchons de palier (43, 44) et la butée fixée au logement (52) est une broche qui est maintenue aux deux extrémités dans des trous dans les deux moitiés de logement (29, 30).

8. Orthèse selon une des revendications 1 à 7, dans laquelle le logement (20) présente à une extrémité une forme partiellement cylindrique (23) avec un grand diamètre et à l'autre extrémité une forme partiellement cylindrique (24) avec un petit diamètre, dans laquelle les deux formes partiellement cylindriques (23, 24) sont connectées ensemble sur le dessous par une paroi de fond plate (25), essentiellement horizontale, du logement (20) et les deux formes partiellement cylindriques sont connectées ensemble sur le dessus par une paroi de recouvrement essentiellement plate (26), inclinée par rapport à l'horizontale, du logement, le corps pivotant (42) est disposé essentiellement dans la région de la forme partiellement cylindrique (23) avec le grand diamètre et l'élément de ressort (51) est disposé essentiellement dans la région de la forme partiellement cylindrique (24) avec le petit diamètre.

9. Orthèse selon une des revendications 1 à 8, dans laquelle l'ouverture de logement (31) s'étend dans les deux moitiés de logement (29, 30).

10. Orthèse selon une des revendications 1 à 9, dans laquelle le logement (20) présente à une extrémité un trou oblong (41) qui s'étend dans les deux moitiés de logement et reçoit une extrémité essentiellement en forme de bande de l'élément de support, dans laquelle l'extrémité en forme de bande (5, 6) de l'élément de support (2) et au moins une moitié de logement (29, 30) présentent des moyens de verrouillage qui coopèrent, lesquels verrouillent l'élément de support (2) avec le logement (20) et peuvent être déverrouillés l'un de l'autre par desserrage des moitiés de logement (29, 30).

11. Orthèse selon les revendications 8 et 10, dans laquelle le trou oblong (41) est réalisé dans l'extrémité du logement (20) avec la grande forme partiellement cylindrique (23).

12. Orthèse selon une des revendications 1 à 11, dans laquelle les moitiés de logement (29, 30) sont connectées ensemble par des moyens de connexion (36 à 39) qui comprennent au moins un trou de vis dans une moitié de logement (29), au moins un filetage interne dans une autre moitié de logement (30) et une vis vissée dans le trou de vis et le filetage interne, et connectant les moitiés de logement ensemble.

13. Orthèse selon une des revendications 1 à 12 qui comprend au moins une broche d'arrêt, et le corps pivotant (42) et le logement (20) de chaque articulation (19) présentent chacun un trou d'arrêt (62) qui s'alignent ensemble dans une position de pivotement déterminée du corps pivotant (42) par rapport au logement (20), dans laquelle la broche d'arrêt peut être insérée sélectivement dans les trous d'arrêt (62) en cas de logement ouvert (20) et fixe le corps pivotant (42) dans la position de pivotement déterminée en cas de logement fermé (20).

14. Orthèse selon une des revendications 1 à 13, dans laquelle le corps pivotant (42), l'élément de connexion (55) et un élément de palier (56) pour les moyens pour le maintien amovible (21) sont réalisés d'une pièce sur une extrémité de l'élément de connexion (55) éloignée du corps pivotant (42).

15. Orthèse selon une des revendications 1 à 14, dans laquelle chaque moyen pour le maintien amovible (21) d'un pied présente une plaque de pied avec des moyens pour la fixation (22) d'un pied ou d'une chaussure.
